# EUROPÄISCHE PATENTSCHRIFT

(11) **EP 3 204 106 B1**
(45) Veröffentlichungstag und Bekanntmachung des Hinweises auf die Patenterteilung: **19.12.2018**
(21) Anmeldenummer: 15777681.6
(22) Anmeldetag: 07.10.2015
(51) Int. Cl.: A61N 1/05

(54) **IMPLANTIERBARE ELEKTRODENANORDNUNG**
IMPLANTABLE ELECTRODE ARRANGEMENT
ENSEMBLE D'ELECTRODES IMPLANTABLE

(30) Priorität: 07.10.2014 DE 102014014943
(43) Veröffentlichungstag der Anmeldung: 16.08.2017
(73) Patentinhaber: Neuroloop GmbH, 79108 Freiburg (DE)
(72) Erfinder: PLACHTA, Dennis, 79279 Vörstetten (DE); GIERTHMÜHLEN, Mortimer, 79104 Freiburg (DE); STIEGLITZ, Thomas, 79110 Freiburg (DE); ZENTNER, Josef, 79117 Freiburg (DE)
(74) Vertreter: Rösler, Uwe
(86) Internationale Anmeldenummer: PCT/EP2015/073132
(87) Internationale Veröffentlichungsnummer: WO 2016/055514

(56) Entgegenhaltungen:
- US-A- 5 215 088
- US-A1- 2012 296 444
- US-A1- 2014 194 950

## Beschreibung

### Technisches Gebiet

Die Erfindung bezieht sich auf eine implantierbare Elektrodenanordnung mit einem zumindest bereichsweise aus biokompatiblen Polymer oder Polyimid bestehenden Trägersubstrat, auf dessen Trägersubstratoberfläche zumindest bereichsweise eine frei zugängliche Elektrodenoberfläche einer auf dem Trägersubstrat aufgebrachten oder in das Trägersubstrat integrierten Elektrode vorhanden ist.

### Stand der Technik

Medizinische Implantate, die zu Zwecken der Zu- und/oder Ableitung von elektrischen Signalen in bzw. von intrakorporal verorteten Bereichen dienen, verfügen zumeist über in biokompatiblen Kunststoffträgern integrierte, metallische Leitungsstrukturen mit frei zugänglichen Elektrodenflächen, die in unmittelbarem Kontakt mit den elektrisch zu stimulierenden bzw. zu überwachenden intrakorporalen Gewebebereichen treten. Das intrakorporale feuchte Milieu, dem medizinische Implantate ausgesetzt sind, stellt hohe Anforderungen an die Materialbeständigkeit sowie auch die Lebensdauer der in oder an einem Trägersubstrat eines Implantats applizierten Elektrodenstrukturen. So besitzen intrakorporale Flüssigkeiten aufgrund ihrer kapillaren Ausbreitungs- und Benetzungseigenschaften vor allem an technischen Oberflächen die Tendenz in kleinste Risse und Zwischenspalte einzudringen, wodurch vor allem auf Trägersubstratoberflächen aufgebrachte Elektrodenstrukturen, die aufgrund des Materialunterschiedes zwischen dem zumeist aus einem biokompatiblen Polymer bestehenden Trägersubstrat und dem metallischen Elektrodenmaterial eine Grenzschicht einschließen, die der kapillar wirkenden Flüssigkeit unentwegt ausgesetzt ist, einer fluid-mechanischen Ablösewirkung unentwegt ausgesetzt sind. Bereits ein sich in der Grenzschicht zwischen Elektrodenstruktur und Trägersubstart ausbildender Mikroriss kann zu einem Ablösen der Elektrodenstruktur von dem polymeren Trägersubstrat führen, wodurch das medizinische Implantat irreversiblen Schaden nehmen kann. Hinzukommt, dass polymere Materialien für molekulares Wasser keine hermetischen Materialien darstellen, weshalb Wasser im Laufe der Zeit auch über rissfreie Oberflächen in polymere Trägermaterialien eines Implantats einzudringen vermögen.

Der Druckschrift DE 10 2011 078 982 A1 ist eine implantierbare Nervenelektrodenanordnung zu entnehmen, bei der Anschlußelektrodenflächen sowie Nerven-Kontaktelektrodenflächen jeweils von einem biokompatiblen, elektrisch isolierenden Substrat umgeben sind, in dem jeweils beide Elektrodenflächen elektrisch miteinander verbindende Leiterbahmen vollständig integriert sind. Die planar und frei zugänglich ausgebildeten Elektrodenflächen sind rahmenartig von einer Ummantelung aus einem mechanisch festen und elektrisch gut isolierenden Polymer umgeben.

Der Druckschrift DE 44 33 111 A1 ist eine Cuff-Elektrode mit einer Vielzahl über ein flexibles, mehrschichtiges aus nichtleitendem Silikon bestehendes Substrat erhabene Elektrodenpins zu entnehmen, die über innerhalb des Substrats verlaufenden Leiterbahnen elektrisch kontaktiert sind. Die Elektrodenpins sind zu Zwecken der Penetration in ein Nervenfaserbündel ausgebildet, um das die Cuff-Elektrode in Form einer peripheren Umwicklung anlegbar ist.

Bei allen bekannten Ausgestaltungen von implantierbaren Elektrodenstrukturen sind keinerlei Maßnahmen getroffen, um dem vorstehenden Problem der Flüssigkeitsbedingten Degradation (Delamination) des Verbundes zwischen Elektrodenkörper und der den Elektrodenkörper umgebenden Trägersubstratmatrix wirksam entgegenzutreten. Weiterhin sind keine Massnahmen beschrieben eine mechanische Festigung des Metall-Polymer-Hybrids in Bereich einer nicht planaren Struktur zu erhöhen.

Aus der Druckschrift US 5, 215,088 ist eine dreidimensionale Elektrodenanordnung mit einer Vielzahl über eine starre Kontaktplattenanordnung erhabenen Elektrodenkontaktsäulen zu entnehmen, die jedoch nicht mit einem biokompatiblen Polymer umgeben sind, weshalb hier das Problem einer möglichen flüssigkeitsbedingten Degradation nicht auftritt.

### Darstellung der Erfindung

Der Erfindung liegt die Aufgabe zugrunde eine implantierbare Elektrodenanordnung mit einem zumindest bereichsweise aus biokompatiblen Polymer oder Polyimid bestehenden Trägersubstrat, auf dessen Trägersubstratoberfläche zumindest bereichsweise eine frei zugängliche Elektrodenoberfläche einer auf dem Trägersubstrat aufgebrachten oder in das Trägersubstrat integrierten Elektroden vorhanden ist, derart weiterzubilden, so dass der vorstehend erläuterte intrakorporal schleichend stattfindende Ablöseprozess zwischen Elektrode und Trägersubstrat zumindest signifikant reduziert oder vollständig vermieden wird.

Die Lösung der der Erfindung zugrunde liegenden Aufgabe ist im Anspruch 1 angegeben. Ferner ist am Anspruch 10 ein lösungsgemäßes Verfahren zur Herstellung der Elektrodenanordnung angegeben. Den Lösungsgedanken in vorteilhafter Weise weiterbildende Merkmale sind Gegenstand der Unteransprüche sowie der weiteren Beschreibung unter Bezugnahme auf die Ausführungsbeispiele zu entnehmen.

Die lösungsgemäße implantierbare Elektrodenanordnung mit einem zumindest bereichsweise aus biokompatiblen Polymer bestehenden Trägersubstrat, auf dessen Trägersubstratoberfläche zumindest bereichsweise eine frei zugängliche Elektrodenoberfläche einer auf dem Trägersubstrat aufgebrachten oder in das Trägersubstrat integrierten Elektrode vorhanden ist, zeichnet sich dadurch aus, dass die Elektrode eine metallische Basisplatte mit einer ebenen Ober- und Unterseite aufweist, an deren Oberseite wenigstens ein, die Oberseite der Basisplatte orthogonal, lokal überragendes Strukturelement vorgesehen ist. Die ebene Oberfläche der metallischen Basisplatte ist parallel zur Trägersubstratoberfläche orientiert angeordnet. Ferner sind die metallische Basisplatte sowie das wenigstens eine, vorzugsweise monolithisch mit der Basisplatte verbundene Strukturelement vollständig von dem biokompatiblen Polymer des Trägersubstarts mittel- und/oder unmittelbar umschlossen, mit Ausnahme eines ersten Oberflächenbereiches des wenigstens einen Strukturelementes, der der Trägersubstratoberfläche abgewandt orientiert ist und der der frei zugänglichen Elektrodenoberfläche entspricht.

Die lösungsgemäße Realisierung einer möglichst dauerstabilen Verbindung zwischen der Elektrode und dem biokompatiblen Polyimid bzw. Polymermaterial des Trägersubstrats spiegelt sich in einer speziellen strukturellen Ausbildungsform der Elektrode wieder sowie in einer dadurch möglichen speziellen Integration der Elektrode in dem Trägersubstrat, wodurch dem intrakorporalen feuchten Milieu bzw. der intrakorporalen Flüssigkeit nur geringfügig Möglichkeit geboten wird in Grenzflächen zwischen dem metallischen Elektrodenmaterial und dem biokompatiblen Polymer des Trägersubstrats einzudringen bzw. diese zu belasten. Hierbei werden die der Feuchtigkeit ausgesetzten Grenzschichtlängen auf ein Minimum reduziert. Dies wird dadurch erreicht, indem der größte Teil des Elektrodenkörpers von dem biokompatiblen Polymer umschlossen ist, lediglich ein kleiner Elektrodenoberflächenbereich bzw. mehrere, klein dimensionierte Elektrodenbereiche ist bzw. sind zu Zwecken der elektrischen Signalübertragung frei an der Trägersubstratoberfläche zugänglich angeordnet und dem intrakorporalen Milieu unter Ausbildung minimaler Grenzschichtlängen ausgesetzt. Sollten im Laufe der Zeit Flüssigkeitsanteile die Grenzschicht degradieren, so dass Flüssigkeit zumindest bereichsweise in die Grenzschichttiefe einzudringen vermag, so verfügt der lösungsgemäß strukturierte Elektrodenkörper über weitere Merkmale, die einen Funktionsverlust des medizinischen Implantats zu vermeiden helfen.

Die Elektrode sieht lösungsgemäß eine metallische, vorzugsweise aus Platin bestehende Basisplatte mit einer ebenen Ober- und Unterseite vor, mit wenigstens einem, vorzugsweise eine Vielzahl die Oberseite der Basisplatte orthogonal lokal überragenden Strukturelementen, die vorzugsweise säulen-, rippen-, hülsen oder stegartig ausgebildet sind. Das wenigstens eine Strukturelement ist monolithisch mit der Basisplatte verbunden und vorzugsweise aus einem über eine sehr hohe elektrische Ladungsübertragungskapazität verfügenden Material gefertigt, vorzugsweise aus Iridium-Oxid. Die metallische Basisplatte ist vollständig von dem biokompatiblen Polymer des Trägersubstrats umschlossen, wie auch das wenigstens eine Strukturelement jedoch mit Ausnahme des ersten Oberflächenbereiches des Strukturelementes, der der Trägersubstratoberfläche abgewandt orientiert ist und diese vorzugsweise nicht überragt.

Somit reduziert sich die frei an der Trägersubstratoberfläche zugängliche Elektrodenkontaktfläche, jedoch ist aufgrund der hermetischen Umschließung der Basisplatte sowie der einstückig daran verbundenen Strukturelemente mit Ausnahme der der Trägersubstratoberfläche abgewandt orientierten Oberflächenbereiche vollständig vom biokompatiblen Polymer des Trägersubstrates umschlossen. Ein Eindringen von milieubedingter Flüssigkeit bzw. Feuchtigkeit zwischen den Elektrodenkörper und dem biokompatiblen Polymer des Trägersubstrates wird somit erheblich erschwert. In einer weiteren bevorzugten Ausführungsform ist vorzugsweise zwischen der Unterseite der metallischen Basisplatte und dem biokompatiblen Polymer des Trägersubstrates zusätzlich eine Haftvermittlerschicht oder eine Haftvermittlerschichtanordnung, bspw. in Form von SiC oder DLC, eingebracht, die sowohl mit dem polymeren Trägersubstrat als auch mit dem metallischen Werkstoff der Elektrode eine kovalente Bindung eingeht. Hierdurch werden die Schichten chemisch miteinander verbunden, wodurch mögliche Mikrokavitäten, die sich für gewöhnlich bei einem direkten Auftrag einer Metallschicht auf einer Polymeroberfläche, bspw. im Wege des Aufsputterns oder Bedampfens ausbilden können, vermieden werden. Gerade derartige Mikrokavitäten können sich aufgrund der Penetrationswirkung von molekularen Wasser in polymeren Materialien im Laufe der Zeit mit Wasser füllen und auf diese Weise zu einem lokalen Ablösen der Metallschicht bzw. Elektrode führen.

Der weiteren Beschreibung unter Bezugnahme auf konkrete, bevorzugte Ausführungsbeispiele kann entnommen werden, dass es sich als vorteilhaft erwiesen hat, zumindest all jene Flächenbereiche der Elektrode mit einer Haftvermittlerschicht zu belegen bzw. zu versehen, die der Trägersubstratoberfläche zumindest abgewandt sind.

In einer weiteren Ausführungsform verfügt das wenigstens eine Strukturelement über wenigstens einen stegartig ausgebildeten Fortsatz, der orthogonal zur Längserstreckung des Strukturelements orientiert und von der Trägersubstratoberfläche beabstandet angeordnet ist, so dass der Fortsatz von dem biokompatiblen Polymer umschlossen ist. Der Fortsatz weist zudem parallel zur Oberseite der Basisplatte orientierte Oberflächenbereiche aufweist, von denen vorzugsweise die der Oberseite der Basisplatte zugewandten Oberflächenbereiche mit einer Haftvermittlerschicht oder einer Haftvermittlerschichtanordnung versehen sind. Selbstverständlich können auch sämtliche vorzugsweise parallel zur Ober- bzw. Unterseite der Basisplatte orientierte, d.h. horizontale Oberflächenbereiche des Elektrodenkörpers mit einer Haftvermittlerschicht oder einer Haftvermittlerschichtanordnung beschichtet sein.

Weitere bevorzugte Ausführungsformen in Bezug auf eine mögliche Ausgestaltung der Elektrodenstruktur werden in Verbindung mit den nachfolgenden Figuren erläutert.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a: detaillierte Darstellung einer in das Trägersubstrat integrierten Elektrode,
- Fig. 1b: alternative Ausbildung eines Strukturelementes und
- Fig. 2: Sequenzbilddarstellungen zur Herstellung der implantierbaren Elektrodenanordnung.

### Wege zur Ausführung der Erfindung, gewerbliche Verwendbarkeit

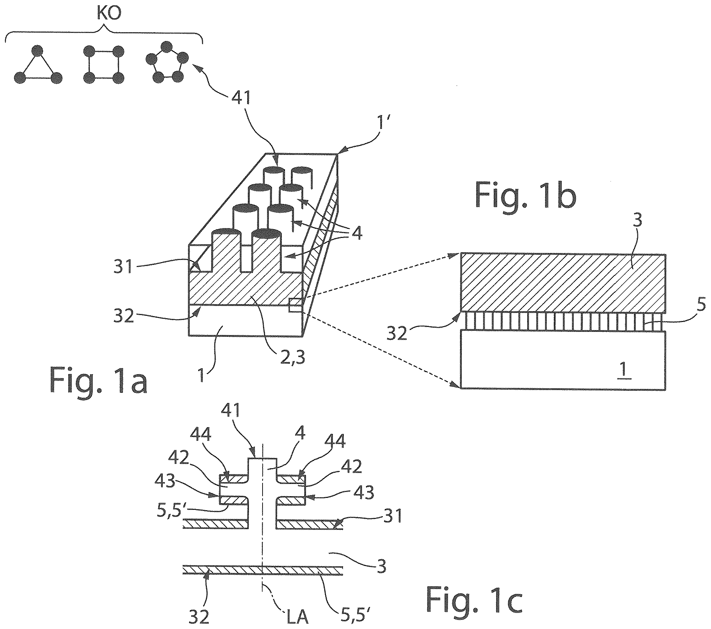

Um die Fügung eines Elektrodenkörpers 2, bspw. in Form eines Elektrodenstreifen, auf oder in einem aus einem biokompatiblen Polymermaterial bestehenden Trägersubstrat 1 dauerhaft zu verbessern, wird vorgeschlagen, den Elektrodenstreifen 3 in der folgenden Weise weitgehend in das Trägersubstrat 1 zu integrieren, siehe hierzu Figur 1a:
Der Elektrodenkörper 2 weist eine metallische Basisplatte 3 auf, die eine Oberseite 31 und eine Unterseite 32 besitzt. Einstückig mit der Oberseite 31 der Basisplatte 3 verbunden sind vorzugsweise über die gesamte Oberseite flächig verteilt, orthogonal erhabene Strukturelemente 4 vorgesehen, vorzugsweise in Form von säulen-, rippen-, steg- oder hülsenartigen Fortsätzen, die über einen der Trägersubstratoberfläche 1' abgewandten Oberflächenbereich 41 verfügen, siehe Fig. 1a, der im implantierten Zustand der Elektrodenanordnung bspw. in unmittelbare Anlage mit dem Epineurium eines Nervenfaserbündels gelangen kann. Zusätzlich ist in vorteilhafter Weise eine Haftvermittlerschicht 5 zumindest zwischen der Unterseite 32 und dem die Basisplatte 3 umgebenden Polymermaterial des Trägersubstrats 1 vorgesehen, siehe Fig. 1b, die eine Detailvergrößerung der in Figur 1a dargestellten implantierbaren Elektrodenanordnung zeigt.

Die Haftvermittlerschicht 3 kann zudem auch auf der Oberseite 31 der Basisplatte 3 aufgebracht sein. Besonders geeignete Haftvermittlerschichten bestehen aus Siliziumcarbid (SiC) sowie Diamond like Carbon (DLC). Vorzugsweise enthalten die Elektrodenkörper 2, insbesondere im Bereich der Strukturelemente 3 Iridium-Oxid, das zu jenen Materialien mit einer der höchsten Ladungsübertragungskapazitäten zu zählen ist.

Die Anzahl sowie auch Anordnung der einzelnen Strukturelemente 4 kann grundsätzlich beliebig gewählt werden, vorzugsweise eignen sich jedoch geometrisch geordnete Konstellationen KO, wie bspw. quadratische, pentagonale, hexagonale oder höherwertige Anordnungsmuster, wie dies aus der Figur 1a zu entnehmen ist.

Eine weitere verbesserte Variante zur Ausbildung des wenigstens einen Strukturelementes 4 ist in Figur 1c illustriert. Figur 1c zeigt den Längsschnitt durch ein Strukturelement 4, das eine orthogonal zur Oberseite 31 der metallischen Basisplatte 3 orientierte Längserstreckung LA aufweist, längs der das Strukturelement 4 wenigstens einen seitlichen Fortsatz 42 vorsieht, der eine orthogonal zur Längsachse LA orientierte Erstreckung besitzt.

Der Fortsatz 42 besitzt einen sogenannte zweiten Oberflächenbereich 43, der parallel zur Oberseite 31 der metallischen Basisplatte 3 und dieser zugewandt orientiert ist. In einer ersten Variante ist auf dem zweiten Oberflächenbereich 43 die Haftvermittlerschicht 5 oder eine Haftvermittlerschichtanordnung 5' aufgebracht, die zudem auch, wie im vorstehenden Fall, auf der Unterseite 32 vorgesehen ist. Der zweite Oberflächenbereich 43 ist vom ersten Oberflächenbereich 31 beabstandet angeordnet und getrennt durch die Haftvermittlerschicht (5) bzw. die Haftvermittlerschichtanordnung (5') vollständig von dem biokompatiblen Polymer umgeben.

In einer zweiten Variante ist die Haftvermittlerschicht (5) bzw. die Haftvermittlerschichtanordnung (5') zusätzlich an der Oberseite 44 des stegartig geformten Fortsatzes 42 und vorzugsweise auch auf der Oberseite 31 der Basisplatte 3 aufgebracht.

Zu Herstellung der lösungsgemäßen, in das biokompatible Polymermaterial eingelassenen, wenigstens einen Elektrode 2 seien die nachfolgenden Prozessschritte unter Bezugnahme auf die Sequenzbilder a bis ab erläutert:
Auf der Oberfläche eines bereitgestellten Siliziumwafers Wa, siehe Schritt a, wird mittels Spin-Coating eine Polyimid-Schicht Pl flächig gleichverteilt aufgebracht, siehe Schritt b. Auf der Polyimid-Schicht Pl wird nachfolgend ein Fotolack Fl, siehe Schritt c, aufgebracht, der mit Hilfe eines Maskenschrittes belichtet und zu Zwecken der Strukturierung des Fotolackes Fl entwickelt wird, siehe Schritt d. Im nächsten Schritt erfolgt die ganzflächige Aufbringung eines Haftvermittlers HV, der beispielsweise mittels einer Bedampfungstechnik abgeschieden wird. Bspw. eignet sich hierzu DLC. Im Schritt f erfolgt eine ganzflächige Metallisation, vorzugsweise mittels Platin Pt, das im Wege eines Sputter- oder Bedampfungsprozesses auf die Haftvermittlerschicht HV abgeschieden wird. Auf diese Weise wird die vorstehend bezeichnete Basisplatte 3 der Elektrode erzeugt. Im nächstfolgenden Schritt g erfolgt ein Lift-Off-Prozess, bei dem sämtliche Materialschichten entfernt werden, mit Ausnahme der strukturierten Platinbasisplatte Pt, die an ihrer Unterseite mit der Haftvermittlerschicht HV auf der Polyimid-Schicht PL aufgebracht ist.

Im Schritt h erfolgt eine erneute Abscheidung einer Haftvermittlerschicht HV. Nachfolgend wird mittels Spin-Coating eine zweite Polyimid-Schicht Pl abgeschieden (siehe Schritt i). In Schritt j erfolgt abermals die Abscheidung einer Fotolack-Schicht Fl mittels Spin-Coating. Nachfolgend wird die Fotolack-Schicht Fl in Schritt k unter Verwendung einer Maske belichtet und entwickelt. Im nachfolgenden Schritt l wird mittels Trockenplasmaätzen die Fotolack-Schicht Fl lokal im Bereich der geöffneten Fotolack-Schicht abgetragen bis hin zur obersten Haftvermittlerschicht.

Im Schritt m wird nun wiederum eine Ir-IrOx Schicht aufgebracht. Im Schritt n erfolgt mittels Lift-Off-Prozess die Extraktion der strukturierten Fotolack-Schicht FL. Im nachfolgenden Schritt o wird eine Fotolack-Schicht FL aufgebracht.
Im Schritt p wird diese Fotolack-Schicht FL entwickelt und auf die Tiefe der Ir-IrOx-Schicht runtergeätzt. Zu beachten ist, dass die Öffnung die Breite der Ir-IrOx-Fläche der Elektrode übersteigt, (siehe Schritt p). Abermals wird eine Haftvermittlerschicht HV aufgebracht, siehe Schritt q. Mittels Aufsputterns bzw. Bedampfens wird in Schritt r eine dritte Metallisation durchgeführt, bei der Iridium Ir sowie vertikal nach oben mit zunehmendem Anteil Iridium-Oxyd IrOx abgeschieden wird. Im anschließenden Schritt s wird erneut eine HV Schicht aufgebracht. Im Verfahrensschritt t erfolgt abermals ein Lift-Off-Prozess, bei dem die auf der Polyimid-Oberfläche aufgebrachte Metallschicht lokal abgetragen wird.

Im Prozessschritt u erfolgt mittels Spin-Coating eine flächige Abscheidung einer Polyimid-Schicht PI. Diese wird im nachfolgenden Schritt v mit einer Fotolack-Schicht Fl überzogen, die im nachfolgend im Schritt w unter Verwendung einer Maske belichtet und entwickelt wird, so dass eine lokale Öffnung innerhalb der Fotolack-Schicht entsteht. Im nachfolgenden Schritt x wird wiederum Ir-IrOx aufgesputtert zur Ausbildung des vorstehend bezeichneten Fortsatzes 42. Im Prozessschritt y erfolgt ein Lift-Off, gefolgt von Schritt z, bei dem eine Fotolack-Schicht Fl mittels Spin Coating aufgebracht wird.

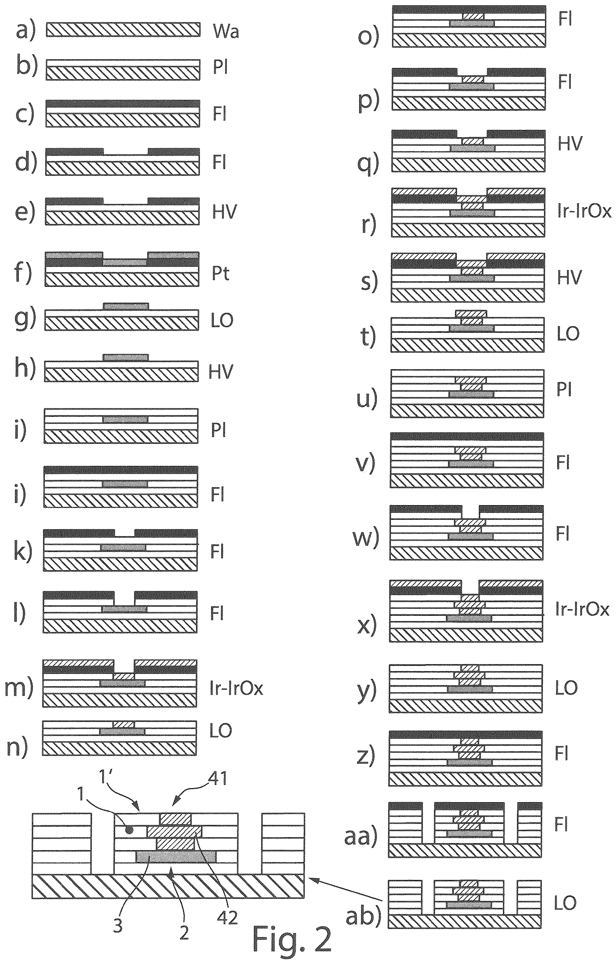

In Schritt aa) wird dieser Fotolack Fl maskenbelegt entwickelt. Mittels Trockenätzen werden zwei Gräben freigelegt, die bis zur Oberfläche des Silizium-Wafers Wa reichen. Im letzten Schritt ab) erfolgt die Beseitigung der Fotolack-Schicht Fl, wodurch eine implantierbare Elektrodenanordnung geschaffen ist, die einen nahezu vollständig vom Polyimid umfassten Elektrodenkörper 2 vorsieht, der eine aus Platin bestehende Basisplatte 3 sowie einen stegartigen Fortsatz 4 besitzt, dessen oberste Elektrodenfläche 41 frei zugänglich ist (siehe vergrößerte Darstellung in Figur 2).

Mit dem vorstehenden Verfahren ist es möglich die Basisplatte mit einer Plattendicke zwischen 10 nm und 5 µm herzustellen, auf der sich Strukturelemente mit einer Länge von 50 nm bis 5 µm erheben.

In einer bevorzugten Anordnung der Basisplatte 3 innerhalb des Trägersubstrats, das sich aus den einzelnen Polyimid-Schichten Pl zusammensetzt, befindet sich die Basisplatte 3 mittig innerhalb des Trägersubstrats, d.h. es gilt die erste Polyimid-Schicht in Schritt a) derart dick auszubilden, dass sie der Gesamtdicke der weiteren Polyimid-Schichten Pl, die in den Schritten i) und u) aufgebracht werden, entspricht. Mit einer derartigen Anordnung der Basisplatte 3 ist der experimentell nachweisbare Vorteil verbunden, dass sich auf die Basisplatte einwirkenden, materialinhärenten Spannungen kompensieren, die sich während eines nachfolgenden Temperprozesses ausbilden. Der Temperprozess ist erforderlich, um eine Materialvorspannung in das Trägersubstrat einzuprägen, durch die sich die implantierbar Manschettenelektrode selbstständig um das Nervenfaserbündel zu wickeln vermag.

### Bezugszeichenliste

- 1: Trägersubstrat
- 1': Trägersubstratoberfläche
- 2: Elektrode
- 3: Basisplatte
- 31: Oberseite
- 32: Unterseite
- 4: Strukturelement
- 41: Erster Oberflächenbereich
- 42: Stegartiger Fortsatz
- 43: Zweiter Oberflächenbereich
- 44: Dritter Oberflächenbereich
- 5: Haftvermittlerschicht
- 5': Haftvermittlerschichtanordnung
- KO: Konstellation
- LA: Längserstreckung

## Patentansprüche

1. Implantierbare Elektrodenanordnung mit einem zumindest bereichsweise aus biokompatiblen Polymer bestehenden Trägersubstrat (1), auf dessen Trägersubstratoberfläche (1') zumindest bereichsweise eine frei zugängliche Elektrodenoberfläche einer auf dem Trägersubstrat (1) aufgebrachten oder in das Trägersubstrat (1) integrierten Elektrode (2) vorhanden ist,
**dadurch gekennzeichnet, dass** die Elektrode (2) eine metallische Basisplatte (3) mit einer ebenen Ober- und Unterseite (31, 32) aufweist, mit wenigstens einem die Oberseite (31) orthogonal, lokal überragenden Strukturelement (4),
dass die ebene Oberfläche der metallischen Basisplatte (4) parallel zur Trägersubstratoberfläche (1') orientiert ist, und
dass die metallische Basisplatte (3) in Verbindung mit dem Strukturelement (4) vollständig von dem biokompatiblen Polymer mittel- und/oder unmittelbar umschlossen ist mit Ausnahme eines ersten Oberflächenbereiches (41) des wenigstens einen Strukturelements (4), der der Trägersubstratoberfläche (1') abgewandt orientiert ist und der frei zugänglichen Elektrodenoberfläche entspricht.

2. Elektrodenanordnung nach Anspruch 1,
**dadurch gekennzeichnet, dass** zumindest zwischen der Unterseite (32) der metallischen Basisplatte (3) und dem biokompatiblen Polymer des Trägersubstrats (1) eine Haftvermittlerschicht (5) oder eine Haftvermittlerschichtanordnung eingebracht ist.

3. Elektrodenanordnung nach Anspruch 1 oder 2,
**dadurch gekennzeichnet, dass** das wenigstens eine Strukturelement (4) einstückig mit der metallischen Basisplatte (3) verbunden ist.

4. Elektrodenanordnung nach einem der Ansprüche 1 bis 3,
**dadurch gekennzeichnet, dass** eine Vielzahl an der Oberseite (31) der metallischen Basisplatte (3) nach einem geometrischen Muster (KO) angeordnete und identisch ausgebildete Strukturelemente (4) vorgesehen ist.

5. Elektrodenanordnung nach einem der Ansprüche 1 bis 4,
**dadurch gekennzeichnet, dass** das wenigstens eine Strukturelement (4) säulen-, rippen-, hülsen oder stegartig ausgebildet ist.

6. Elektrodenanordnung nach einem der Ansprüche 1 bis 5,
**dadurch gekennzeichnet, dass** das wenigstens eine Strukturelement (4) eine orthogonal zur Oberseite (31) der metallischen Basisplatte (3) orientierte Längserstreckung (LA) aufweist, längs der das Strukturelement (4) wenigstens einen orthogonal zur Längserstreckung (LA) orientierten, stegartigen Fortsatz (42) aufweist, der wenigstens einen zweiten Oberflächenbereich (43) vorsieht, der parallel zur Oberseite (31) der metallischen Basisplatte (3) orientiert ist und auf dem die Haftvermittlerschicht (5) oder eine Haftvermittlerschichtanordnung aufgebracht ist, und
dass der zweite Oberflächenbereich (43) vom ersten Oberflächenbereich (41) beabstandet angeordnet und mittelbar vollständig von dem biokompatiblen Polymer umgeben ist.

7. Elektrodenanordnung nach Anspruch 6,
**dadurch gekennzeichnet, dass** der zweite Oberflächenbereich (43) der Trägersubstratoberfläche (1') abgewandt orientiert ist.

8. Elektrodenanordnung nach einem der Ansprüche 1 bis 7,
**dadurch gekennzeichnet, dass** die Basisplatte (3) eine Plattendicke zwischen 10 nm und 5 µm und das wenigstens eine Strukturelement (4) eine sich über die Basisplatte (3) erhabene Länge von 50 nm bis 5 µm aufweist.

9. Elektrodenanordnung nach einem der Ansprüche 1 bis 8,
**dadurch gekennzeichnet, dass** das Trägersubstrat (1) eine orthogonal zur Trägersubstratoberfläche (1') orientierte Substratdicke aufweist, und dass die Basisplatte (3) mittig zur Substratdicke angeordnet ist.

10. Verfahren zur Herstellung der implantierbaren Elektrodenanordnung nach einem der Ansprüche 1 bis 9,
**gekennzeichnet durch** folgende Verfahrensschritte:
- Aufbringen einer metallischen Basisplatte (3) auf einem aus biokompatiblen Polymer bestehenden Trägersubstrat (1),
- Monolithisches Aufbringen wenigstens eines sich orthogonal über die Oberseite (31) der Basisplatte (3) erstreckenden Strukturelementes (4) und
- Aufbringen von biokompatiblen Polymer auf das Trägersubstrat (1) und die Basisplatte (3) in Verbindung mit dem Strukturelement (4) derart, dass das wenigstens eine Strukturelement (4) mit Ausnahme eines ersten Oberflächenbereiches (41) des wenigstens einen Strukturelements (4), der der Oberseite (31) der Basisplatte (3) abgewandt orientiert ist, vollständig von dem biokompatiblen Polymer des Trägersubstrats (1) mittel- und/oder unmittelbar umschlossen werden.

11. Verfahren nach Anspruch 10,
**dadurch gekennzeichnet, dass** die metallische Basisplatte (3) sowie das wenigstens eine Strukturelement (4) im Wege eines Aufdampf- oder Sputterprozesses abgeschieden werden.

## Claims

1. An implantable electrode configuration having a carrier substrate (1) made of a biocompatible polymer in at least some areas, a freely accessible electrode surface of an electrode (2) applied to this carrier substrate (1) or integrated into the carrier substrate (1) being present in at least some areas on its carrier substrate surface (1'),
**characterized in that** the electrode (2) has a metallic base plate (3) with a planar top side (31) and bottom side (32), with at least one locally protruding structural element (4) arranged orthogonally to the top side (31); the planar surface of the metallic base plate (3) being oriented in parallel with the carrier substrate surface (1'), and
that the metallic base plate (3) in combination with the structural element (4) are directly and/or indirectly enclosed by the biocompatible polymer, except for a first surface area (41) of the at least one structural element (4), which is oriented so that it turns away the carrier substrate surface (1') and corresponds to the freely accessible electrode surface.

2. The electrode configuration according to claim 1,
**characterized in that** an adhesion promoter layer (5) or an adhesion promoter layer configuration is introduced at least between the bottom side (32) of the metallic base plate (3) and the biocompatible polymer of the carrier substrate (1).

3. The electrode configuration according to claim 1 or 2,
**characterized in that** the at least one structural element (4) is connected in one piece to the metallic base plate (3).

4. The electrode configuration according to any one of claims 1 to 3,
**characterized in that** a plurality of structural elements (4) designed to be identical and arranged on the top side (31) of the metallic base plate (3) according to a geometric pattern (KO) is provided.

5. The electrode configuration according to any one of claims 1 to 4,
**characterized in that** the at least one structural element (4) is designed in the form of columns, ribs, sleeves or webs.

6. The electrode configuration according to any one of claims 1 to 5,
**characterized in that** the at least one structural element (4) has a longitudinal extent (LA) oriented orthogonally to the top side (31) of the metallic base plate (3), the structural element (4) having at least one web-type protrusion (42) oriented orthogonally along the longitudinal extent (LA) and provides at least one second surface area (43) being oriented parallel to the top side (31) of the metallic base plate (3) and to which the adhesion promoter layer (5) or an adhesion promoter layer configuration applied thereto, and
that the second surface area (43) is arranged at a distance from the first surface area (41) and is surrounded indirectly completely by the biocompatible polymer.

7. The electrode configuration according to claim 6,
**characterized in that** the second surface area (43) is oriented to turn away from the carrier substrate surface (1').

8. The electrode configuration according to any one of claims 1 to 7,
**characterized in that** the base plate (8) has a plate thickness between 10 nm and 5 µm, and the at least one structural element (4) has an elevated length of 50 nm to 5 µm above the base plate (3).

9. The electrode configuration according to any one of claims 1 to 8,
**characterized in that** the carrier substrate (1) has a substrate thickness oriented orthogonally to the carrier substrate surface (1'), and the base plate (3) is arranged centrally with respect to the substrate thickness.

10. A method for producing the implantable electrode configuration according to any one of claims 1 to 9, **characterized by** the following method steps:
- applying a metallic base plate (3) to a carrier substrate (1) made of a biocompatible polymer,
- monolithic application of at least one structural element (4) extending orthogonally over the top side (31) of the base plate (3), and
- applying biocompatible polymer to the carrier substrate (1) and the base plate (3) in combination with the structural element (4) in such a way that the at least one structural element (4) except for a first surface area (41) of the at least one structural element (4), which is oriented so that it turns away from the top side (31) of the base plate (3), is directly and/or indirectly enclosed by the biocompatible polymer of the carrier substrate (1).

11. The method according to claim 10,
**characterized in that** the metallic base plate (3) and the at least one structural element (4) are deposited by a vapor deposition or sputtering process.

## Revendications

1. Agencement implantable d'électrodes, pourvu d'un substrat porteur (1), constitué au moins par endroits d'un polymère biocompatible, sur la surface de substrat porteur (1') duquel est présente au moins par endroits une surface d'électrode librement accessible d'une électrode (2) appliquée sur le substrat porteur (1) ou intégrée dans le substrat porteur (1), **caractérisé en ce que** l'électrode (2) comporte une plaque de base (3) métallique, comprenant une face supérieure et une face inférieure (31, 32) planes, avec au moins un élément de structure (4) saillant localement de manière orthogonale par-dessus la face supérieure (31), **en ce que** la surface plane de la plaque de base (4) métallique est orientée à la parallèle de la surface de substrat porteur (1') et **en ce que** la plaque de base (3) métallique en association avec l'élément de structure (4) est entièrement entourée, directement et/ou indirectement par le polymère biocompatible, à l'exception d'une première zone de surface (41) de l'au moins un élément de structure (4), qui est orientée à l'opposée de la surface de substrat porteur (1') et qui correspond à la surface d'électrode librement accessible.

2. Agencement d'électrode selon la revendication 1, **caractérisé en ce qu'**au moins entre la face inférieure (32) de la plaque de base (3) métallique et du polymère biocompatible du substrat porteur (1) est intégré(e) une couche d'agent d'adhérence (5) ou un agencement d'agent adhérence.

3. Agencement d'électrode selon la revendication 1 ou 2, **caractérisé en ce que** l'au moins un élément de structure (4) est assemblé en monobloc avec la plaque de base (3) métallique.

4. Agencement d'électrode selon l'une quelconque des revendications 1 à 3, **caractérisé en ce qu'**il est prévu une pluralité d'éléments de structure (4) placés sur la face supérieure (31) de la plaque de base (3) métallique selon un motif géométrique (KO) et conçus à l'identique.

5. Agencement d'électrode selon l'une quelconque des revendications 1 à 4, **caractérisé en ce que** l'au moins un élément de structure (4) est conçu sous la forme de colonnes, de nervures, de douilles ou de barrettes.

6. Agencement d'électrode selon l'une quelconque des revendications 1 à 5, **caractérisé en ce que** l'au moins un élément de structure (4) présente une extension longitudinale (LA) orientée de manière orthogonale par rapport à la face supérieure (31) de la plaque de base (3) métallique, le long de laquelle l'élément de structure (4) comporte au moins un prolongement (42) en forme de barrette, orienté de manière orthogonale par rapport à l'extension longitudinale (LA), qui prévoit au moins une deuxième zone de surface (43) qui est orientée à la parallèle de la face supérieure (31) de la plaque de base (3) métallique et sur laquelle est appliqué(e) la couche d'agent d'adhérence (5) ou l'agencement d'agent adhérence et **en ce que** la deuxième zone de surface (43) est placée avec un écart par rapport à la première zone de surface (41) est entourée directement et totalement par le polymère biocompatible.

7. Agencement d'électrode selon la revendication 6, **caractérisé en ce que** la deuxième zone de surface (43) de la surface de substrat porteur (1') est orientée à l'opposée.

8. Agencement d'électrode selon l'une quelconque des revendications 1 à 7, **caractérisé en ce que** la plaque de base (3) présente une épaisseur de plaque comprise entre 10 nm et 5 µm et **en ce que** l'au moins un élément de structure (4) présente une longueur 50 nm à 5 µm, en relief sur la plaque de base (3).

9. Agencement d'électrode selon l'une quelconque des revendications 1 à 8, **caractérisé en ce que** le substrat porteur (1) présente une épaisseur de substrat orientée de manière orthogonale par rapport à la surface de substrat porteur (1') et **en ce que** la plaque de base (3) est placée au centre de l'épaisseur de substrat.

10. Procédé, destiné à la fabrication de l'agencement implantable d'électrode selon l'une quelconque des revendications 1 à 9, **caractérisé par** les étapes de procédé suivantes, consistant à :
- appliquer une plaque de base (3) métallique sur un substrat porteur (1) composé d'un polymère biocompatible,
- appliquer de manière monolithique au moins un élément de structure (4) s'étendant de manière orthogonale par-dessus la face supérieure (31) de la plaque de base (3) et
- appliquer du polymère biocompatible sur le substrat porteur (1) et la plaque de base (3), en association avec l'élément de structure (4) de sorte que l'au moins un élément de structure (4) à l'exception d'une première zone de surface (41) de l'au moins un élément de structure (4), qui est orientée à l'opposée de la face supérieure (31) de la plaque de base (3) soit entouré totalement, directement et/ou indirectement par le polymère biocompatible du substrat porteur (1) .

11. Procédé selon la revendication 10, **caractérisé en ce que** la plaque de base (3) métallique, ainsi que l'au moins un élément de structure (4) sont déposés par voie d'un processus métallisation ou de vaporisation.
